# EUROPEAN PATENT SPECIFICATION

(11) **EP 3 609 417 B1**
(45) Date of publication and mention of the grant of the patent: **09.06.2021**
(21) Application number: 18717037.8
(22) Date of filing: 10.04.2018
(51) Int. Cl.: A61B 18/04, H05H 1/48, C01B 3/04, A61B 18/00, A61L 2/00, A61L 2/14

(54) **DEVICE FOR HUMAN MEDICAL AND VETERINARY TREATMENT**
VORRICHTUNG ZUR HUMANMEDIZINISCHEN UND VETERINÄRMEDIZINISCHEN BEHANDLUNG
DISPOSITIF DE TRAITEMENT MÉDICAL HUMAIN ET VÉTÉRINAIRE

(30) Priority: 11.04.2017 DE 102017003526
(43) Date of publication of application: 19.02.2020
(73) Proprietor: Lohmann&Rauscher GmbH, 1140 Wien (AT)
(72) Inventor: BUCHEGGER, Patricia, 2801 Katzelsdorf (AT); HARREITHER, Wolfgang, 2514 Traiskirchen (AT)
(74) Representative: Seranski, Klaus
(86) International application number: PCT/EP2018/059152
(87) International publication number: WO 2018/189174

(56) References cited:
- KR-A- 20160 072 759
- US-A1- 2003 139 734
- US-A1- 2006 189 976
- US-A1- 2013 199 540
- US-A1- 2016 106 993

## Description

The invention relates to a device for human medical or veterinary treatment as specified in claim 1 Preferred embodiments of the invention are disclosed in the dependent claims.

In therapeutic treatment, in addition to conventional pharmaceutical products and physical treatment approaches, such as for example compression and/or negative pressure therapy, more recently treatment approaches have also been used in which reactive gases are used. Corresponding treatment approaches have become known in the field of dermatology for the treatment of dermatoses and wounds of any type, of oncological diseases and in dentistry. Over the course of plasma treatment reactive gas species are formed and are enriched specifically over the treatment area. This constitutes one possible physical treatment approach.

Reactive gases contain highly reactive components. These display their oxidative potential on the surface where they oxidise proteins, lipids and nucleic acids non-selectively. Higher eukaryotic cells have highly developed defence mechanisms and can deal with the oxidative stress that is caused substantially better than bacteria, fungi or viruses. These defence mechanisms include, among other things, antioxidants, enzymes that destroy ROS (Reactive Oxygen Species), such as catalases or dismutases, and DNA repair machinery. Therefore, reactive gases can be successfully used in particular in the treatment of wounds and dermatoses.

During plasma treatment the reactive gases are generally formed by transferring sufficient energy in a gas discharge. In this type of gas discharge a plasma composed of partially or fully charged particles is produced, from which reactive gas species develop. Plasma is often generated in electrostatic or electromagnetic fields, e.g. by alternating or direct current excitation or microwave excitation. For regenerative medical purposes cold atmospheric pressure plasma are generally used. If air is used as the reaction or process gas, reactive oxygen and nitrogen species, such as e.g. ozone (O₃) and hydrogen peroxide (H₂O₂) or nitric oxides with a strongly oxidizing effect are predominantly produced as products of the plasma. In addition, electrons and ions as well as photons emitted upon relaxation of the excited plasma components are produced in the plasma itself, which may themselves display an antiseptic effect that assists with wound healing.

Cold atmospheric pressure plasma can be formed by electric barrier discharge between insulation-coated electrodes by microwave excitation or a piezoelectric transformer as an active dielectric in a gas-filled space. In plasma therapy a basic distinction is made between the following treatment approaches:

### 1. Direct plasma therapy

In this therapy approach the plasma is generated in the treatment or wound area itself, generally by means of dielectric barrier discharge, the treatment area or the skin being able to be used as the electrode or counter-electrode in the plasma generation by means of gas discharge. Devices for direct plasma treatment are described, for example, in WO 2011/023478 and WO 2015/184395.

### 2. Indirect plasma therapy

In indirect plasma therapy the plasma is generated in a special plasma generator and conveyed by diffusion or optionally by means of compressed air, a pump or a ventilator to the treatment area. With the device for indirect plasma therapy described in DE 10 2012 003 563 A1 the reactive gases are generated in a plasma generator which is disposed in a housing. With the aid of a flow generator disposed within the housing the reactive gases are conveyed in the form of a free jet to the treatment area.

Further devices and methods for plasma therapy are disclosed in US 2013/199540 A1, US 2016/106993 A1, US 2003/139734 A1, KR 2016 0072759 A and US 2006/189976 A1.

With the conventional devices and methods for indirect plasma therapy it has been shown that in many cases satisfactory treatment results cannot be achieved.

In view of these problems in the prior art, the object underlying the invention is to provide devices for indirect plasma therapy with which improved therapy results can be achieved.

With regard to the device, this object is achieved by a further development of the known devices as specified in claim 1.

With devices according to the invention the turbulent flow in the reaction chamber and/or the gas discharge zone leads to a buffer region being formed therein in which the individual molecules of the process gases are, as it were, intermediately stored, and over the duration of the intermediate storage are influenced by the electromagnetic fields of the gas discharge. The yield of reactive gases is thus increased by influencing the gas discharge. As a result, this leads to improved therapy success.

It has proven to be particularly advantageous if the outlet opening of the reaction chamber has a smaller opening area in a plane running perpendicular to the main flow direction of the gas flow generated by the flow generator than an inlet opening of the reaction chamber and/or the discharge zone. This gas flow aligned to the outlet opening then gives rise to dynamic pressure before the outlet opening. This dynamic pressure in turn leads to a turbulent flow guiding the process gases at least partially back into the discharge zone and creates the aforementioned buffer for the process gases by the process gases furthermore being subjected to the influence of the discharge.

In terms of the creation of defined flow ratios it has proven to be advantageous if the reaction chamber has a peripheral wall that is, at least in sections, in the form of a circular cylindrical jacket and at one end of which an inlet opening for the process gases can be provided, and at the other end of which the outlet opening is located. The cylinder axis of the peripheral wall section made in the form of a circular cylindrical jacket preferably runs approximately parallel to the main flow direction of the gas flow generated by the flow generator.

In terms of providing a simple and reliable application device it has proven to be advantageous if the outlet opening of the reaction chamber passes through a connecting piece made in the form of a tube coupling and preferably running approximately parallel to the peripheral wall in the form of a circular cylindrical jacket, to which connecting piece an application tube of the application device can be fitted.

In terms of avoiding an excessively great flow resistance while at the same time ensuring a sufficiently turbulent flow by means of which the buffering of the process gases can be ensured to a sufficient degree, it has proven to be advantageous if the ratio of the opening area of the inlet opening or the area of the gas discharge zone in a direction running perpendicular to the main flow direction to the opening area of the outlet opening is greater than 5, preferably greater than 10, in particular assuming a value of 15 or more, but is less than 90, in particular less than 50, preferably 40 or less.

It has been shown empirically that the diameter of the reaction chamber is smaller than 20 mm, preferably smaller than 15 mm, but greater than 5 mm, preferably greater than 7.5 mm, in particular approximately 10 mm. If the diameter of the reaction chamber is too large, the influence of the process gases in the buffer zone formed by the reaction chamber becomes too small due to the gas discharge, while at the same time an undesired abreaction of the reactive gases may already take place in the reaction chamber. On the other hand, a diameter of the reaction chamber that is too small leads to an undesired pressure increase in the reaction chamber, and this may lead to excessive collisional quenching of the reactive gases.

For the reasons given above, it is also advantageous if the reaction chamber has a length of 15 mm or more, in particular 20 mm or more in the main flow direction of the gas flow generated by the flow generator, the length of the reaction chamber, however, preferably being less than 80 mm, in particular less than 60 mm. A reaction chamber length of approximately 40 mm has proven to be particularly advantageous.

In terms of the effective production of reactive gases it has furthermore proven to be advantageous if the ratio of the axial length of the reaction chamber to the maximum diameter of the reaction chamber is greater than 2, in particular greater than 3, but smaller than 10, in particular smaller than 5.

Within the framework of the invention the discharge zone may also be located at least partially within the reaction chamber. For this purpose a discharge electrode, optionally encased with a dielectric or a piezotransformer may be located in the reaction chamber as a dielectric electrode.

The reaction chamber itself is preferably made of a chemically inert material, such as for example of electrically insulating plastic. In devices according to the invention the discharge direction and/or the discharge type may be specifically steered by the integration of one or more dielectric and/or metal parts. For example, it is conceivable to locate at least one metal part in the region of an inner boundary surface of the reaction chamber. The use of a dielectric barrier in the region of the discharge electrode leads to dielectric barrier discharge, whereas e.g. a metal ring after the discharge zone may lead to an arc discharge in this direction. In terms of influencing the composition of the reactive gases it has proven to be particularly advantageous if a humidifying device is provided for humidifying gases in the discharge zone and/or in the flow direction before the discharge zone and/or in the flow direction after the discharge zone.

As already explained above in connection with the use of an outlet opening that passes through a connecting piece, according to the invention the application device has a tube, in particular a gas-tight plastic tube. Due to the flexibility of this type of tube the reactive gases generated in the discharge zone and/or the reaction chamber can be brought to almost any application locations. In order to avoid undesired collisional quenching of the reactive gases during transportation by the application device , according to the invention the ratio of the length to the internal diameter of the tube is more than 7, preferably more than 10 and/or less than 30, in particular less than 25. The ratio of the length of the tube to the diameter is chosen to correspond to the diameter of the outlet opening and the dynamic pressure that is set so that a laminar flow forms in the tube in which there is only a small collision probability for the reactive gases, as a result of which the collisional quenching can be largely suppressed. According to the invention, the tube has an internal diameter of the range of between 2 and 8 mm, in particular of approximately 4 mm.

As already explained above in connection with devices according to the invention, a method according to the invention for generating reactive gases that can be used in plasma therapy, in which reactive gases are generated in a discharge zone from process gases, in particular air, and are discharged in the direction of an outlet opening of a reaction chamber, is essentially characterised in that a turbulent gas flow is generated in the discharge zone and/or the reaction chamber.

Within the framework of generating a turbulent gas flow a dynamic pressure in the range between 5 and 50 Pa, preferably between 10 and 40 Pa, in particular 23 to 35 Pa is generated here.

A compromise between the enrichment of reactive gases on the one hand and the particle flow of reactive gases on the other hand is achieved if the gases are discharged from the reaction chamber at a flow speed of less than 20 l/min, preferably less than 10 I/min, but more than 2.5 l/min. With excessively slow discharge of the reactive gases from the reaction chamber the reactive gases are enriched particularly well, but the particle flow of reactive gases becomes so small overall that successful therapy can no longer be achieved to a sufficient degree. Furthermore, when a minimum flow speed of 2.5 l/min is set, one can regularly dispense with the use of additional cooling devices for the elements bringing about the plasma generation, such as for example electrodes or piezo transformers. On the other hand, excessively rapid discharge of reactive gases from the reaction chamber leads to an overly small concentration of reactive gases in the gas that is discharged, which in turn puts the therapy success at risk. For these reasons the aforementioned upper and lower limits for the flow speed are particularly advantageous.

Within the framework of methods according to the invention the composition of the reactive gases can be influenced if the process gases are humidified before, during and/or after the discharge zone. Air can particularly preferably be used as the process gas. The humidification of the process gases advantageously takes place such that the relative humidity of the process gases is lowered to less than 50 %, in particular less than 15 %. With strong humidification of the process gases, hydrogen peroxide is predominantly formed as the reactive gas, but with a concentration that is too high, this abreacts with water and oxygen. For this reason the relative humidity of the process gases is limited to less than 50 %. Within the framework of the invention it has proven to be advantageous if the ambient air has been dried to a value of 10% relative humidity before being introduced into the discharge zone.

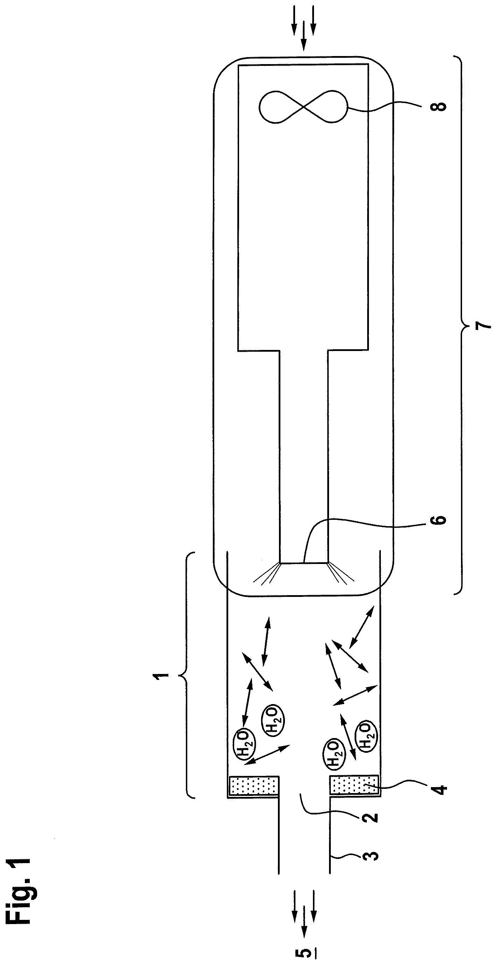

The single figure of the drawing shows a schematic illustration of a device according to the invention. The device comprises a ventilator 8, with the aid of which air is conveyed into the region of an electrode 6. Gas discharge with plasma generation takes place in the region of the electrode 6. Connected downstream of the electrode 6 is a reaction chamber 1 in which the process gases are buffered and from which the process gases are discharged through an outlet opening 2. The reaction chamber is delimited by a wall in the form of a circular cylindrical jacket to the end of which facing away from the electrode 6 a front wall is attached in which a connecting piece 3 for a tube that can be used as an application device is attached. The internal diameter of the connecting piece 3 is smaller than the internal diameter of the wall of the reaction chamber 1 that is in the form of a circular cylindrical jacket. In this way a dynamic pressure can form on the front surface of the reaction chamber 1 facing away from the electrode 6, which pressure in turn causes a turbulent flow by means of which the process gases pass a number of times into the discharge region, i.e. into the region of the electrode 6.

In the embodiment of the invention illustrated by the drawing, an air humidification device made in the form of a pad is provided in the region of the front surface of the reaction chamber 1 facing away from the electrode 6, by means of which the composition of the reactive gases can be influenced. In the embodiment of the invention illustrated by the drawing a gas discharge zone forms in the region of the electrode 6, which zone reaches into the reaction chamber 1.

The invention is not restricted to the embodiment illustrated by the drawing. In a further development the use of piezoelectric transformers is also conceivable. With these transformers the greatest potential is at the corners on the front end. Therefore, discharge and plasma formation normally take place at these corners. The integration of metal parts into the reaction chamber before the gas discharge zone may additionally bring about a discharge along the edges. These additional parasitic discharges may increase the efficiency of the ionisation of the ambient air because more plasma can be produced in a shorter time.

### List of reference numbers

- 1: ionisation chamber
- 2: outlet constriction
- 3: tube coupling
- 4: pad for air humidity enrichment
- 5: multiply ionised reactive gas
- 6: plasma generation
- 7: plasma source
- 8: ventilator
- 9: input gas

## Claims

1. A device for human medical or veterinary treatment comprising a gas discharge generator (7) designed to generate reactive gases that can be used in plasma therapy in a gas discharge zone,
a flow generator (8) designed to generate a flow of gas from the gas discharge zone through a reaction chamber (1) in the direction of an outlet opening (2) of the reaction chamber (1), and
an application device coupled to the outlet opening (2) for discharging the reactive gases from the reaction chamber (1) to the application location,
wherein the ratio of the opening area of an inlet opening of the reaction chamber (1) to the opening area of the outlet opening is greater than 5, and the ratio of the opening area of the inlet opening of the reaction chamber (1) to the opening area of the outlet opening (2) is less than 90, the flow generator (7) and the reaction chamber (1) are designed to generate a turbulent flow in the reaction chamber and/or the gas discharge zone wherein the diameter of the reaction chamber (1) is smaller than 20 mm, and greater than 5 mm, and in that the application device has a gas-tight plastic tube wherein the ratio of the length to the internal diameter of the tube is more than 7 and less than 30, and the tube has an internal diameter in the range between 2 and 8 mm.

2. The device according to Claim 1, wherein the reaction chamber (1) has a peripheral wall that is, at least in sections, in the form of an approximately circular cylindrical jacket, and at one end of which can be an inlet opening of the reaction chamber (1) and at the other end of which the outlet opening (2) is located.

3. The device according to any of the preceding claims, wherein the outlet opening (2) passes through a connecting piece made in the form of a tube coupling (3) and preferably running approximately parallel to the peripheral wall in the form of a circular cylindrical jacket.

4. The device according to any of the preceding claims, wherein the reaction chamber (1) has a length of 15 mm or more, in particular 20 mm or more in the main flow direction of the gas flow generated by the flow generator (8), but less than 80 mm, in particular less than 60 mm, the length of the reaction chamber (1) preferably being approximately 40 mm.

5. The device according to any of the preceding claims, wherein the ratio of the axial length of the reaction chamber (1) to the maximum diameter is greater than 2, in particular greater than 3, but is smaller than 10, in particular is smaller than 5.

6. The device according to any of the preceding claims, wherein the discharge zone is located at least partially within the reaction chamber (1).

7. The device according to any of the preceding claims, wherein the reaction chamber (1) is made, at least in sections, of insulating plastic.

8. The device according to any of the preceding claims, wherein at least one metal part is located in the region of an inner boundary surface of the reaction chamber (1).

9. The device according to any of the preceding claims, wherein the gas discharge generator (6) can be operated to generate a dielectric barrier discharge.

10. The device according to any of the preceding claims, wherein the device further comprises a humidifying device (4) for humidifying process gases in the discharge zone and/or in the flow direction before the discharge zone and/or in the flow direction after the discharge zone.

## Patentansprüche

1. Vorrichtung zur humanmedizinischen oder veterinärmedizinischen Behandlung, die Folgendes umfasst: einen Gasentladungsgenerator (7) zum Erzeugen von in der Plasmatherapie einsetzbaren reaktiven Gasen in einer Gasentladungszone,
einen Strömungsgenerator (8) zum Erzeugen einer Gasströmung von der Gasentladungszone durch eine Reaktionskammer (1) auf eine Auslassöffnung (2) der Reaktionskammer (1), und eine an die Auslassöffnung (2) gekoppelte Applikationseinrichtung zum Ableiten der reaktiven Gase aus der Reaktionskammer (1) zum Applikationsort,
wobei das Verhältnis der Öffnungsfläche einer Einlassöffnung der Reaktionskammer (1) zur Öffnungsfläche der Auslassöffnung größer als 5 ist und das Verhältnis der Öffnungsfläche der Einlassöffnung der Reaktionskammer (1) zur Öffnungsfläche der Auslassöffnung (2) kleiner als 90 ist, und wobei der Strömungsgenerator (8) und die Reaktionskammer (1) zum Erzeugen einer turbulenten Strömung in der Reaktionskammer und/oder der Gasentladungszone ausgelegt sind,
wobei der Durchmesser der Reaktionskammer (1) kleiner als 20 mm und größer als 5 mm ist, und wobei die Applikationseinrichtung einen gasdichten Kunststoffschlauch aufweist, wobei das Verhältnis von Länge zum Innendurchmesser des Schlauchs größer als 7 und kleiner als 30 ist, und wobei der Schlauch einen Innendurchmesser im Bereich zwischen 2 und 8 mm aufweist.

2. Vorrichtung nach Anspruch 1, wobei die Reaktionskammer (1) eine zumindest abschnittweise etwa kreiszylindermantelförmig ausgeführte Umfangswand aufweist, an deren einem Ende eine Einlassöffnung der Reaktionskammer (1) sein kann und an deren anderem Ende die Auslassöffnung (2) angeordnet ist.

3. Vorrichtung nach einem der vorhergehenden Ansprüche, wobei die Auslassöffnung (2) einen als Schlauchkopplung (3) ausgeführten und vorzugsweise etwa parallel zur kreiszylindermantelförmigen Umfangswand verlaufenden Anschlussstutzen durchsetzt.

4. Vorrichtung nach einem der vorhergehenden Ansprüche, wobei die Reaktionskammer (1) eine Länge in Hauptströmungsrichtung der mit dem Strömungsgenerator (8) erzeugten Gasströmung von 15 mm oder mehr, insbesondere 20 mm oder mehr aufweist, aber weniger als 80 mm, insbesondere weniger als 60 mm aufweist, wobei die Länge der Reaktionskammer (1) vorzugsweise etwa 40 mm beträgt.

5. Vorrichtung nach einem der vorhergehenden Ansprüche, wobei das Verhältnis der axialen Länge der Reaktionskammer (1) zum maximalen Durchmesser größer als 2 ist, insbesondere größer als 3 ist, aber kleiner als 10 ist, insbesondere kleiner als 5 ist.

6. Vorrichtung nach einem der vorhergehenden Ansprüche, wobei die Entladungszone zumindest teilweise in der Reaktionskammer (1) angeordnet ist.

7. Vorrichtung nach einem der vorhergehenden Ansprüche, wobei die Reaktionskammer (1) zumindest abschnittweise aus isolierendem Kunststoff ausgeführt ist.

8. Vorrichtung nach einem der vorhergehenden Ansprüche, wobei im Bereich einer inneren Begrenzungsfläche der Reaktionskammer (1) mindestens ein Metallteil angeordnet ist.

9. Vorrichtung nach einem der vorhergehenden Ansprüche, wobei der Gasentladungsgenerator (6) zum Erzeugen einer dielektrisch behinderten Entladung betreibbar ist.

10. Vorrichtung nach einem der vorhergehenden Ansprüche, wobei die Vorrichtung ferner eine Befeuchtungseinrichtung (4) zum Befeuchten von Prozessgasen in der Entladungszone und/oder in Strömungsrichtung vor der Entladungszone und/oder in Strömungsrichtung nach der Entladungszone umfasst.

## Revendications

1. Dispositif de traitement médical humain ou vétérinaire comprenant un générateur de décharge de gaz (7) conçu pour générer des gaz réactifs, susceptibles d'être utilisés dans les thérapies par plasma gazeux, dans une zone de décharge de gaz,
un générateur de flux (8) conçu pour générer un flux de gaz partant de la zone de décharge de gaz et passant par une chambre de réaction (1) en direction d'un orifice de sortie (2) de la chambre de réaction (1), et
un dispositif d'application couplé à l'orifice de sortie (2) pour décharger les gaz réactifs de la chambre de réaction (1) vers le site d'application ;
dans lequel dispositif le rapport entre la surface d'ouverture d'un orifice d'entrée de la chambre de réaction (1) et la surface d'ouverture de l'orifice de sortie est supérieur à 5, et le rapport entre la surface d'ouverture de l'orifice d'entrée de la chambre de réaction (1) et la surface d'ouverture de l'orifice de sortie (2) est inférieur à 90, et le générateur de flux (8) et la chambre de réaction (1) sont conçus pour générer un flux turbulent dans la chambre de réaction et/ou la zone de décharge de gaz, et
dans lequel dispositif le diamètre de la chambre de réaction (1) est inférieur à 20 mm, et supérieur à 5 mm, le dispositif d'application possédant un tube en plastique étanche aux gaz, le rapport entre la longueur et le diamètre intérieur du tube étant supérieur à 7 et inférieur à 30, et le tube présentant un diamètre intérieur compris entre 2 et 8 mm.

2. Dispositif selon la revendication 1, dans lequel la chambre de réaction (1) possède une paroi périphérique sous la forme, au moins par sections, d'une enveloppe approximativement cylindrique circulaire, dont une extrémité peut présenter un orifice d'entrée de la chambre de réaction (1) et à l'autre extrémité de laquelle est situé l'orifice de sortie (2).

3. Dispositif selon l'une quelconque des revendications précédentes, dans lequel l'orifice de sortie (2) traverse une pièce de raccordement qui est réalisée sous la forme d'un raccord à tube (3) et de préférence orientée approximativement parallèle à la paroi périphérique sous forme d'enveloppe cylindrique circulaire.

4. Dispositif selon l'une quelconque des revendications précédentes, dans lequel la chambre de réaction (1) présente une longueur faisant 15 mm ou plus, notamment 20 mm ou plus dans le sens principal du flux de gaz généré par le générateur de flux (8), mais inférieure à 80 mm, notamment inférieure à 60 mm ; la longueur de la chambre de réaction (1) faisant de préférence environ 40 mm.

5. Dispositif selon l'une quelconque des revendications précédentes, dans lequel le rapport entre la longueur axiale de la chambre de réaction (1) et le diamètre maximal est supérieur à 2, notamment supérieur à 3, mais est inférieur à 10, notamment inférieur à 5.

6. Dispositif selon l'une quelconque des revendications précédentes, dans lequel la zone de décharge est située au moins partiellement dans la chambre de réaction (1).

7. Dispositif selon l'une quelconque des revendications précédentes, dans lequel la chambre de réaction (1) est fabriquée, au moins par sections, dans du plastique isolant.

8. Dispositif selon l'une quelconque des revendications précédentes, dans lequel au moins une partie métallique est située dans la région d'une surface limite intérieure de la chambre de réaction (1).

9. Dispositif selon l'une quelconque des revendications précédentes, dans lequel le générateur de décharge de gaz (6) peut être mis en œuvre pour générer une décharge à barrière diélectrique.

10. Dispositif selon l'une quelconque des revendications précédentes, dans lequel le dispositif comprend en outre un dispositif humidificateur (4) destiné à humidifier les gaz de processus dans la zone de décharge et/ou en amont de la zone de décharge, vu dans le sens du flux, et/ou en aval de la zone de décharge, dans le sens du flux.
